# EUROPÄISCHE PATENTSCHRIFT

(11) **EP 2 780 315 B1**
(45) Veröffentlichungstag und Bekanntmachung des Hinweises auf die Patenterteilung: **24.01.2018**
(21) Anmeldenummer: 12787018.6
(22) Anmeldetag: 16.11.2012
(51) Int. Cl.: C07C 69/30, C09D 4/00

(54) **VERWENDUNG VON GLYCEROLDIESTERN ALS REAKTIVVERDÜNNER UND DIESE ENTHALTENDE BESCHICHTUNGSMITTEL**
USE OF GLYCEROL DIESTERS AS REACTIVE DILUENTS AND COATING COMPOSITIONS CONTAINING THEM
UTILISATION DES DIESTERS DE GLYCEROL EN TANT QUE DILUANT RÉACTIF, ET COMPOSITIONS DE REVÊTEMENT LES CONTENANT

(30) Priorität: 17.11.2011 US 201161560842 P; 17.11.2011 EP 11189618
(43) Veröffentlichungstag der Anmeldung: 24.09.2014
(73) Patentinhaber: BASF Coatings GmbH, 48165 Münster (DE)
(72) Erfinder: HOFFMANN, Peter, 48308 Senden (DE); PORCHER, Sebastien, 67063 Ludwigshafen (DE); GOUDARD, Melanie, F-13006 Marseille (FR)
(74) Vertreter: BASF IP Association
(86) Internationale Anmeldenummer: PCT/EP2012/072884
(87) Internationale Veröffentlichungsnummer: WO 2013/072481

(56) Entgegenhaltungen:
- EP-A1- 0 908 479
- WO-A1-00/17179
- WO-A2-2007/074333
- US-A- 4 423 071
- ROUZEAU, S. ET AL.: "Criteria for the preparation of cross-linked poly(meth)acrylate microparticles by solution free radical polymerization", EUROPEAN POLYMER JOURNAL, PERGAMON PRESS LTD. OXFORD, GB, Bd. 43, Nr. 10, 28. September 2007 (2007-09-28), Seiten 4398-4414, XP022277530, ISSN: 0014-3057, DOI: 10.1016/J.EURPOLYMJ.2007.07.015
- VAN DIJK, J.H. ET AL.: "Analysis of coatings additives and free monomers using liquid and capillary gas chromatography", PROCEEDINGS XVITH FAT-IPEC CONGRESS, 9-14 MAY 1982, LIÈGE,, 1982, pages 253-265, XP009165723,

## Beschreibung

Die vorliegende Erfindung betrifft die Verwendung von Glyceroldiestern als Reaktivverdünner sowie Beschichtungsmittelzusammensetzungen enthaltend derartige Glyceroldiester. Des Weiteren betrifft die Erfindung Mehrschichtlackierungen die unter Verwendung der Beschichtungsmittelzusammensetzungen erhalten werden und ein Verfahren zur Herstellung derartiger Mehrschichtlackierungen und damit lackierte Substrate.

Es besteht die stete Nachfrage an Lacken, die umweltfreundlich sind und weniger Lösemittel freisetzen. Insbesondere besteht ein zunehmender Bedarf an lösemittelarmen Beschichtungsmitteln. Eine Möglichkeit den Lösemittelgehalt in Beschichtungsstoffen zu senken besteht darin Lösemittel auszuwählen, die eine besonders gut verdünnende, das heißt viskositätssenkende Wirkung besitzen. Diese Möglichkeit wird häufig mit dem Einsatz niedermolekularer, d.h. in der Regel weniger viskoser Bindemittel verbunden. Trotz dieser Entwicklung sind niedrige Gehalte an flüchtigen organischen Verbindungen ("Volatile Organic Compounds"; kurz VOC) nur schwer ohne eine Beeinträchtigung des Lackerscheinungsbilds ("Appearance") zu erreichen.

Eine weitere Möglichkeit Beschichtungsmittel mit einem geringen VOC-Gehalt zu erhalten ist der Einsatz sogenannter Reaktivverdünner. Hierbei handelt es sich um Lösemittel, die durch chemische Reaktion mit zumindest einem im gehärteten Lack verbleibenden Bindemittel oder Vernetzungsmittel in die Lackschicht eingebaut werden. Der VOC-Gehalt wird somit nicht erhöht.

Die EP 0 908 479 A1 offenbart Polyurethan-Zweikomponentensysteme, die cyclische Acetale und/oder Ketale als Reaktivverdünner enthalten.

Die WO 2007/074333 A2 betrifft Beschichtungsmittelzusammensetzungen enthaltend mindestens ein Bindemittel und einen Reaktivverdünner, der eine Viskosität von 0,5 Pa s bei 20 °C besitzt, wobei der Reaktivverdünner durch Reaktion wenigstens eines Polyols mit wenigstens einer Dicarbonsäure oder einem Alkylester oder Anhydrid derselben und wenigstens einer mehrfach ungesättigten Fettsäure oder einem Alkylester derselben, erhältlich ist.

Eine der Aufgaben der vorliegenden Erfindung war es Reaktivverdünner zu finden, die nicht nur eine gute verdünnende Wirkung besitzen, sondern auch die Appearance des gehärteten Lacks verbessern und für einen guten Verlauf ("Levelling") sorgen.

Diese Aufgabe wurde durch die Verwendung von Glyceroldiestern der allgemeinen Formel (I) 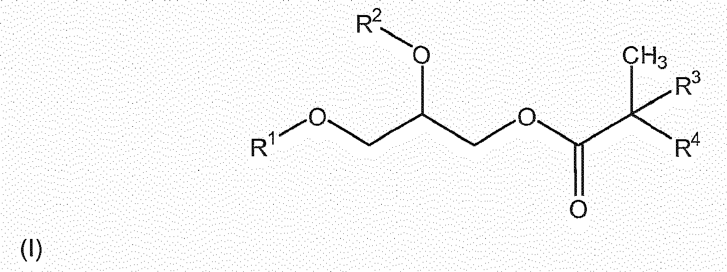 worin einer der beiden Reste R¹ oder R² für Wasserstoff steht und der nicht für Wasserstoff stehende der beiden Reste R¹ und R² für einen Rest 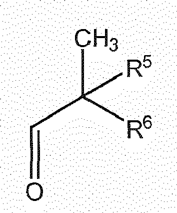 steht und die Reste R³, R⁴, R⁵ und R⁶ unabhängig voneinander für einen Alkylrest mit 1 bis 10 Kohlenstoffatomen stehen, mit der Maßgabe, dass die Reste R³ und R⁴ gemeinsam mindestens 4 Kohlenstoffatome enthalten und die Reste R⁵ und R⁶ gemeinsam mindestens 4 Kohlenstoffatome enthalten, als Reaktivverdünner in Beschichtungsmittelzusammensetzungen, Klebstoffen und Dichtungsmitteln, gelöst.

Vorzugsweise beträgt die gemeinsame Anzahl der Kohlenstoffatome in den Resten R³ und R⁴ bzw. die gemeinsame Anzahl der Kohlenstoffatome in den Resten R⁵ und R⁶ maximal 18, besonders bevorzugt 4 bis 10 und ganz besonders bevorzugt 4 bis 8. Bei den Resten R³, R⁴, R⁵ und R⁶ handelt es sich um Alkylreste mit 1 bis 10, besonders bevorzugt 1 bis 8 und ganz besonders bevorzugt 1 bis 6 Kohlenstoffatomen. Diese Alkylreste können substituiert oder unsubstituiert sein und sind vorzugsweise unsubstituiert. Diese Alkylreste können linear oder verzweigt sein.

Sind diese Alkylreste substituiert, so sind als Substituenten vorzugsweise ein oder mehrere Reste gewählt aus der Gruppe bestehend aus Hydroxylgruppen, O(CO)ₙR⁹-Gruppen mit n = 0 oder 1 und R⁹ = verzweigtes oder unverzweigtes Alkyl mit 1 bis 6 Kohlenstoffatomen und Ether- und/oder Estergruppen enthaltenden aliphatischen Resten mit 1 bis 10, vorzugsweise 2 bis 10 Kohlenstoffatomen, vorhanden.

Diese Verbindungen der allgemeinen Formel (I) werden im Folgenden als Glyceroldiester" bezeichnet.

Erhältlich sind die Glyceroldiester der oben angegebenen Formel (I) durch Umsetzung einer Verbindung der allgemeinen Formel (II) 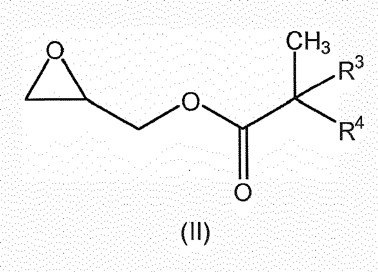 mit einer Verbindung der allgemeinen Formel (III)

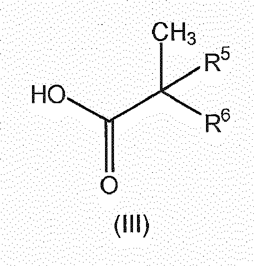

Die Umsetzung erfolgt durch ringöffnende Addition der COOH-Gruppe der Verbindung der Formel (III) an die Epoxygruppe der Verbindung der Formel (II). Die Reste R³, R⁴, R⁵ und R⁶ sind wie oben zum Glyceroldiester beschrieben definiert.

Die Ringöffnung kann unter Bildung einer primären Hydroxylgruppe oder einer sekundären Hydroxylgruppe erfolgen. Wird eine primäre Hydroxylgruppe erhalten, so ist in den Verbindungen der allgemeinen Formel (I) R¹ = Wasserstoff und der Rest R² ein Rest der Formel O=C-C(CH₃)(R⁵)(R⁶). Bei Erhalt einer sekundären Hydroxylgruppe ist in den Verbindungen der allgemeinen Formel (I) R² = Wasserstoff und R¹ ein Rest der Formel O=C-C(CH₃)(R⁵)(R⁶).

In der Regel entstehen als Reaktionsprodukt Mischungen aus Verbindungen der allgemeinen Formel (I), wobei ein Teil der Produkte primäre Hydroxylgruppen trägt und der andere Teil sekundäre Hydroxylgruppen aufweist. Das Verhältnis primärer zu sekundärer Hydroxylgruppen lässt sich durch die Reaktionsbedingungen, insbesondere die Reaktionstemperatur und den Einsatz von Katalysatoren beeinflussen. Ohne Einsatz eines Katalysators überwiegt in der Regel der Anteil an Verbindungen mit primären Hydroxylgruppen. Beim Einsatz von beispielsweise Ethyltriphenylphosphoniumiodid als Katalysator kann das Verhältnis sekundär zu primär erhöht werden.

Durch Steuerung des Verhältnisses von primären zu sekundären Hydroxylgruppen ist es möglich die Reaktivität des Reaktivverdünners in einem gewissen Umfang zu beeinflussen. Da primäre Hydroxylgruppen enthaltende Verbindungen der allgemeinen Formel (I) im Allgemeinen schneller reagieren als solche mit sekundären Hydroxylgruppen, lässt sich durch die Reaktionsführung bei der Herstellung der Verbindungen der allgemeinen Formel (I) deren Reaktivität, beispielsweise gegenüber Isocyanatgruppen, steuern. Diese vorteilhafte Flexibilität des Verfahrens eröffnet die Möglichkeit maßgeschneiderte erfindungsgemäße Glyceroldiester zu erhalten und so die Topfzeit der Beschichtungsmittel, welche die erfindungsgemäßen Glyceroldiester enthalten, zu beeinflussen.

Die Verbindungen der allgemeinen Formel (II) lassen sich beispielsweise durch Umsetzung von Epichlorhydrin mit einer Carbonsäure R³R⁴(CH₃)C-COOH wie in EP 1 115 714 B1 (Beispiel 1) beschrieben, erhalten. Ein besonders bevorzugt einsetzbares Umsetzungsprodukt, welches unter die allgemeine Formel (II) fällt, ist der unter der Handelsbezeichnung Cardura® E10 erhältliche Glycidylester der Versatic®-Säure.

Carbonsäuren R³R⁴(CH₃)C-COOH und somit auch Carbonsäuren der analog definierten allgemeinen Formel (III) sind käuflich erhältlich. Unter diesen besonders bevorzugt sind die unter der Bezeichnung Versatic®-Säuren bekannten stark verzweigten, gesättigten Monocarbonsäuren mit längeren Seitenketten und tertiären COOH-Gruppen, die beispielsweise durch Kochsche Carbonsäuresynthese aus Olefinen, Kohlenmonoxid und Wasser entstehen. Ganz besonders bevorzugt ist unter diesen die Neodecansäure.

Die Glyceroldiester lassen sich beispielsweise herstellen indem die Verbindungen der allgemeinen Formeln (II) und (III) in einem Lösemittel vorgelegt werden und auf eine Temperatur im Bereich von 100 bis 160°C erhitzt werden. Bei Verwendung leicht siedender Lösemittel kann die Reaktion unter erhöhtem Druck durchgeführt werden. Der Reaktionsfortschritt wird mittels Bestimmung der Säurezahl verfolgt. Als Lösemittel geeignet sind beispielsweise aromatische Kohlenwasserstoffe wie Xylol, Toluol, Solvent Naphtha, Ester wie Butylacetat, Pentylacetat, Etherester wie z. B. Methoxypropylacetat, Ethoxiethylpropionat, Ketone wie Methylethylketon, Methylisoamylketon und Methylisobutylketon. Die so erhaltenen erfindungsgemäßen Glyceroldiester können dann, sofern erwünscht nach (teilweiser) Abtrennung des Lösemittels, in den erfindungsgemäßen Beschichtungsmittelzusammensetzungen eingesetzt werden.

Die Glyceroldiester können jedoch auch *in-situ,* das heißt ohne deren Isolierung, vor oder während der Synthese der polymeren Polyole (a), insbesondere des hydroxylgruppenhaltigen Poly(meth)acrylats gebildet werden. Dies kann beispielsweise durch Vorlegen der Verbindungen der allgemeinen Formeln (II) und (III) in einem geeigneten Lösemittel, Erhitzen auf eine Temperatur von beispielsweise 80 bis 180°C, zum Beispiel 160°C und nachfolgendem Zudosieren der das polymere Polyol (a) bildenden Monomere und Katalysatoren erfolgen. Geeignete Katalysatoren sind, sofern es sich beim polymeren Polyol (a) um ein hydroxylgruppenhaltiges Poly(meth)acrylat handelt, beispielsweise Peroxidkatalysatoren, wie zum Beispiel Di-tert.-Butylperoxid (DTBP). Bei dieser Ausführungsform können nur Monomermischungen eingesetzt werden, die nur untergeordnete Anteile (< 10 Gew.-% bezogen auf das Gesamtgewicht aller Monomere) an Säure- bzw. Epoxidfunktionellen Monomeren beinhalten. Die Reaktionsführung kann unter Normaldruck oder Überdruck erfolgen. Der Reaktionsmischung können vor, während oder nach der Polymerisation geeignete Lösemittel zugesetzt werden, um einerseits die Polymerisationreaktion, andererseits die resultierende Viskosität zu beeinflussen. Vor, während oder nach der Polymerisation können geringe Mengen (0,01 -2,0 Gew.-% bezogen auf die Gesamtmenge des festen Polymers) eines geeigneten Reduktionsmittels zugesetzt werden, um besonders helle Harzlösungen zu erhalten. Als Reduktionsmittel kommen bevorzugt Alkylphosphite zum Einsatz, besonders bevorzugt wird Triisodecylphosphit eingesetzt.

Gegenstand der vorliegenden Erfindung ist die Verwendung der Glyceroldiester als Reaktivverdünner in Beschichtungsmittelzusammensetzungen, Klebstoffen und Dichtstoffen. Die Beschichtungsmittelzusammensetzungen, Klebstoffe und Dichtstoffe enthalten dabei vorzugsweise Verbindungen mit einer oder mehreren gegenüber den Hydroxylgruppen des Glyceroldiesters reaktiven Gruppen, wie beispielsweise Isocyanatgruppen oder den reaktiven Gruppen an Aminoplastharzen oder Triazinen wie TACT. Vorzugsweise sind die gegenüber den Hydroxylgruppen reaktiven Verbindungen in den Beschichtungsmittelzusammensetzungen, Klebstoffen und Dichtstoffen sogenannte Vernetzungsmittel und/oder Bindemittel. Besonders bevorzugt werden die Glyceroldiester in Beschichtungsmittelzusammensetzungen, insbesondere Decklacken, und ganz besonders bevorzugt Klarlacken als Reaktivverdünner verwendet. Hierbei entfalten sie nicht nur eine viskositätssenkende Wirkung, sondern führen in der Regel zu Beschichtungen mit einem verbesserten Verlauf. Ganz besonders bevorzugt ist daher die Verwendung der Glyceroldiester als Reaktivverdünner und Verlaufmittel.

Weiterer Gegenstand der Erfindung ist eine Beschichtungsmittelzusammensetzung, enthaltend
(a) mindestens ein polymeres Polyol gewählt aus der Gruppe bestehend aus Poly(meth)acrylat-Polyolen, Polyester-Polyolen, Polyurethan-Polyolen und Polysiloxan-Polyolen,
(b) mindestens ein aliphatisches Polyisocyanat und
(c) mindestens einen Glyceroldiester der allgemeinen Formel (I) 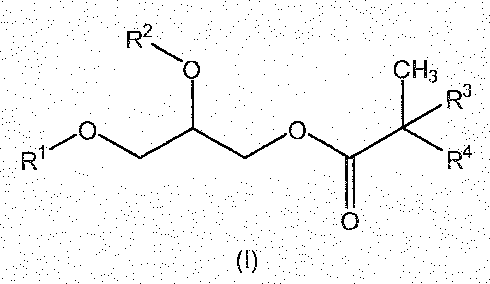 worin die Reste R¹, R², R³ und R⁴ wie in Anspruch 1 definiert sind.

Bevorzugte Definitionen der Reste R³, R⁴, R⁵ und R⁶ finden sich oben zum Glyceroldiester.

Im Folgenden wird die erfindungsgemäße Beschichtungsmittelzusammensetzung auch kurz als "erfindungsgemäßes Beschichtungsmittel" bezeichnet.

Unter einem "polymeren Polyol" ist hierin ein Polyol mit mindestens zwei Hydroxylgruppen zu verstehen, wobei vom Begriff "polymer" hierin auch der Begriff "oligomer" umfasst ist. Oligomere bestehen hierin aus mindestens drei Monomereinheiten.

Die bevorzugten polymeren Polyole (a) weisen gewichtsmittlere Molekulargewichte M_{w} > 500 Dalton, gemessen mittels GPC (Gelpermeationschromatographie) gegen einen Polystyrolstandard, bevorzugt von 800 bis 100000 Dalton, insbesondere von 1000 bis 50000 Dalton auf. Höchst bevorzugt sind solche mit einem gewichtsmittleren Molekulargewicht von 1000 bis 10000 Dalton.

Die polymeren Polyole (a) weisen bevorzugt eine Hydroxylzahl (OH-Zahl) von 30 bis 400 mg KOH/g, insbesondere von 100 bis 300 mg KOH/g, und ganz besonders bevorzugt 120 bis 180 mg KOH/g auf.

In einer besonders bevorzugten Ausführungsform der erfindungsgemäßen Beschichtungsmittelzusammensetzung weicht die OH-Zahl des polymeren Polyols (a) oder der Mischung der polymeren Polyole (a) um nicht mehr als 20 % und ganz besonders bevorzugt um nicht mehr als 10 % von der OH-Zahl der in der Beschichtungsmittelzusammensetzung eingesetzten Glyceroldiester-Komponente (c) ab.

Die Glasübergangstemperaturen der polymeren Polyole (a), gemessen per DSC (Differential-Thermoanalyse, DSC 1000 der Fa TA-Instruments der Waters GmbH, Eschborn, Aufheizrate 10°C/Minute) liegen bevorzugt zwischen -150 und 100°C, besonders bevorzugt zwischen -120°C und 80°C.

Unter dem Begriff "Poly(meth)acrylat" werden sowohl Polyacrylate als auch Polymethacrylate sowie Polymere, die sowohl Methacrylate bzw. Methacrylsäure als auch Acrylate bzw. Acrylsäure enthalten verstanden. Die Poly(meth)acrylate können neben Acrylsäure, Methacrylsäure und/oder den Estern von Acrylsäure und/oder Methacrylsäure auch weitere ethylenisch ungesättigte Monomere enthalten. Vorzugsweise sind die Monomere aus welchen die Poly(meth)acrylate erhalten werden monoethylenisch ungesättigte Monomere. Unter einem "Poly(meth)acrylat-Polyol" wird ein Poly(meth)acrylat verstanden, welches mindestens zwei Hydroxylgruppen enthält.

Anstelle oder zusätzlich zu den Poly(meth)acrylat-Polyolen können auch Polyester-Polyole eingesetzt werden. Hierbei ist ein Polyester-Polyol ein Polyester, der mindestens zwei Hydroxylgruppen trägt.

Bei einem gemeinsamen Einsatz von Poly(meth)acrylat-Polyolen und Polyester-Polyolen können beide Komponenten einzeln hergestellt werden oder indem das Poly(meth)acrylat-Polyol in einer Polyester-Polyol-Komponente oder deren Lösung in geeignetem Lösemittel *in situ* polymerisiert wird.

Unter den polymeren Polyolen ganz besonders bevorzugt sind Polyacrylat-Polyole und/oder Polymethacrylat-Polyole sowie deren Mischpolymerisate. Diese können einstufig oder mehrstufig hergestellt werden. Sie können beispielsweise als statistische Polymere, Gradientencopolymere, Blockcopolymere oder Pfropfpolymere vorliegen.

Die erfindungsgemäß ganz besonders bevorzugten Poly(meth)acrylat-Polyole sind in der Regel Copolymerisate mit anderen vinylisch ungesättigten Monomeren und weisen vorzugsweise gewichtsmittlere Molekulargewichte M_{w} von 1000 bis 20000 g/mol, insbesondere von 1500 bis 10000 g/mol auf, jeweils gemessen mittels Gelpermeationschromatographie (GPC) gegen einen Polystyrolstandard.

Die Glasübergangstemperatur der Poly(meth)acrylat-Polyole liegt in der Regel zwischen -100 und 100°C, insbesondere zwischen -50 und 80°C (gemessen mittels DSC-Messungen, wie oben angegeben).

Die Poly(meth)acrylat-Polyole weisen bevorzugt eine OH-Zahl von 60 bis 250 mg KOH/g, insbesondere 70 bis 200 mg KOH/g und besonders bevorzugt 120 bis 180 mg KOH/g auf. Ihre Säurezahl beträgt vorzugsweise 0 bis 30 mg KOH/g.

Die Hydroxylzahl gibt an, wieviel mg Kaliumhydroxid der Essigsäure-Menge äquivalent sind, die von 1 g Substanz bei der Acetylierung gebunden wird. Die Probe wird bei der Bestimmung mit Essigsäureanhydrid-Pyridin gekocht und die entstehende Säure mit Kaliumhydroxidlösung titriert (DIN 53240-2).

Die Säurezahl gibt hierin die Anzahl der mg Kaliumhydroxid an, die zur Neutralisation von 1 g der jeweiligen Verbindung verbraucht wird (DIN EN ISO 2114).

Als hydroxylgruppenhaltige Monomerbausteine der Poly(meth)acrylat-Polyole werden bevorzugt ein oder mehrere Hydroxyalkyl(meth)acrylate, wie insbesondere 2-Hydroxyethylacrylat, 2-Hydroxyethylmethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat sowie insbesondere 4-Hydroxybutylacrylat und/oder 4-Hydroxybutylmethacrylat, eingesetzt. Insbesondere lassen sich auch durch die industrielle Herstellung bedingte Gemische vorteilhaft einsetzen. So besteht beispielsweise industriell hergestelltes Hydroxypropylmethacrylat aus etwa 20 - 30% 3-Hydroxypropylmethacrylat und 70 - 80% 2-Hydroxypropylmethacrylat.

Als weitere Monomerbausteine werden für die Synthese der Poly(meth)acrylat-Polyole bevorzugt Alkyl(meth)acrylate eingestetzt, wie vorzugsweise Ethylacrylat, Ethylmethacrylat, Propylacrylat, Propylmethacrylat, Isopropylacrylat, Isopropylmethacrylat, Butylacrylat, Butylmethacrylat, Isobutylacrylat, Isobutylmethacrylat, tert-Butylacrylat, tert-Butylmethacrylat, Amylacrylat, Amylmethacrylat, Hexylacrylat, Hexylmethacrylat, Ethylhexylacrylat, Ethylhexylmethacrylat, 3,3,5-Trimethylhexylacrylat, 3,3,5-Trimethylhexylmethacrylat, Stearylacrylat, Stearylmethacrylat, Laurylacrylat oder Laurylmethacrylat, Cycloalkylacrylate und/oder Cycloalkylmethacrylate, wie Cyclopentylacrylat, Cyclopentylmethacrylat, Isobornylacrylat, Isobornylmethacrylat oder insbesondere Cyclohexylacrylat und/oder Cyclohexylmethacrylat.

Als weitere Monomerbausteine für die Synthese der Poly(meth)acrylatpolyole können vinylaromatische Kohlenwasserstoffe, wie Vinyltoluol, alpha-Methylstyrol oder insbesondere Styrol, Amide oder Nitrile der Acryl- oder Methacrylsäure, Vinylester oder Vinylether, sowie in untergeordneten Mengen insbesondere Acryl- und/oder Methacrylsäure eingesetzt werden.

Geeignete Polyester-Polyole sind beispielsweise in EP-A-0 994 117 und EP-A-1 273 640 beschrieben. Insbesondere lassen sich geeignete Polyester-Polyole wie dem Durchschnittsfachmann bekannt ist durch Polykondensation aus Polyolen und Polycarbonsäuren oder deren Anhydriden erhalten.

Als in der Polykondensationsreaktion einsetzbares Polyol oder einsetzbare Polyolmischung eignen sich insbesondere mehrwertige Alkohole bzw. deren Mischungen, wobei die Alkohole wenigstens zwei, vorzugsweise wenigstens drei Hydroxylgruppen aufweisen. Bevorzugt enthält die eingesetzte Polyolmischung oder ist das eingesetzte Polyol wenigstens ein höherfunktionelles Polyol, das wenigstens drei Hydroxylgruppen aufweist. Geeignete höherfunktionelle Polyole mit wenigstens drei Hydroxylgruppen sind vorzugsweise ausgewählt aus der Gruppe bestehend aus Trimethylolpropan (TMP), Trimethylolethan (TME), Glycerin, Pentaerythrit, Zuckeralkoholen, Ditrimethylolpropan, Dipentaerythrit, Diglycerin, Trishydroxyethylisocyanurat und Mischungen davon. In einer speziellen Ausführungsform besteht das zur Herstellung der Polyester-Polyole eingesetzte Polyol nur aus höherfunktionellen Polyolen mit mehr als drei Hydroxylgruppen. In einer weiteren speziellen Ausführungsform enthält die zur Herstellung der Polyester-Polyole eingesetzte Polyolmischung wenigstens ein höherfunktionelles Polyol mit mindestens drei Hydroxylgruppen und wenigstens ein Diol. Geeignete Diole sind zum Beispiel Ethylenglykol, Propylenglykol, Butandiol-1,4, Pentandiol-1,5, Hexandiol-1,6, Neopentylglykol, 2-Butyl-2-ethyl-propandiol-1,3, Diethylenglykol, Dipropylenglykol, höhere Polyetherdiole, Dimethylolcyclohexan, und Mischungen der vorgenannten Polyole.

Zur Herstellung der Polyester-Polyole geeignete Polycarbonsäuren oder deren Anhydride sind beispielsweise Phthalsäure, Phthalsäureanhydrid, Isophthalsäure, Terephthalsäure, Trimellithsäure, Trimellithsäureanhydrid, Pyromellithsäure, Pyromellithsäuredianhydrid, Tetrahydrophthalsäure, 1,2-, 1,3- oder 1,4-Cyclohexandicarbonsäure, 1,2-Cyclohexandicarbonsäureanhydrid, Tricyclodecandicarbonsäure, Bernsteinsäure, Glutarsäure, Adipinsäure, Pimelinsäure, Korksäure, Azelainsäure, Sebacinsäure, Maleinsäure, Maleinsäureanhydrid, Fumarsäure, Itaconsäure, Mesaconsäure, Citraconsäure, Dimerfettsäuren und Mischungen davon. In einer bevorzugten Ausführungsform werden zur Herstellung der in der vorliegenden Erfindung eingesetzten Polyester-Polyole ausschließlich Polycarbonsäuren der nachfolgend genannten Klasse 1 eingesetzt. Die Klasse 1 besteht aus Phthalsäure, Phthalsäureanhydrid, Isophthalsäure, Terephthalsäure, Trimellithsäure, Trimellithsäureanhydrid, Pyromellithsäure, Pyromellithsäuredianhydrid, Tetrahydrophthalsäure, Tetrahydrophthalsäureanhydrid, Hexahydrophthalsäure, Hexahydrophthalsäureanhydrid und Mischungen davon.

In einer weiteren bevorzugten Ausführungsform werden zur Herstellung der in der vorliegenden Erfindung eingesetzten Polyester-Polyole mindestens 50 Gew.-%, bezogen auf das Gesamtgewicht der Polycarbonsäure-Komponente an Polycarbonsäuren oder deren Anhydriden aus Klasse 1 eingesetzt. Nach dieser Ausführungsform besteht die Polycarbonsäure-Komponente zu höchstens 50 Gew.-%, bezogen auf ihr Gesamtgewicht, aus wenigstens einer Polycarbonsäure der nachfolgend aufgeführten Klasse 2 bestehend aus Bernsteinsäure, Glutarsäure, Adipinsäure, Pimelinsäure, Korksäure, Azelainsäure, Sebacinsäure, Dimerfettsäuren und Mischungen davon. Nach dieser Ausführungsform kann die Polycarbonsäure-Komponente neben wenigstens einer Polycarbonsäure der Klasse 1 und gegebenenfalls wenigstens einer Polycarbonsäure der Klasse 2, zusätzlich noch bis zu höchstens 10 Gew.-% wenigstens einer Polycarbonsäure der Klasse 3 bestehend aus Maleinsäure, Maleinsäureanhydrid, Fumarsäure, Itaconsäure, Mesaconsäure, Citraconsäure und Mischungen davon, enthalten.

Geeignete Polyurethan-Polyole werden wie dem Durchschnittsfachmann bekannt vorzugsweise durch Umsetzung von Polyesterpolyol-Präpolymeren - zum Beispiel auch solchen der oben genannten Art - mit geeigneten Di- oder Polyisocyanaten hergestellt und sind beispielsweise in der EP-A-1 273 640 beschrieben.

Geeignete Polysiloxan-Polyole sind beispielsweise in der WO-A-01/09260 beschrieben, wobei die dort angeführten Polysiloxan-Polyole bevorzugt in Kombination mit weiteren Polyolen, insbesondere solchen mit höheren Glasübergangstemperaturen, zum Einsatz kommen.

Sind in der erfindungsgemäßen Beschichtungsmittelzusammensetzung noch weitere Bindemittel, außer den unter den Begriff der polymeren Polyole (a) subsumierbaren Bindemittel enthalten, so können diese mit den anderen Komponenten des Beschichtungsmittels reagieren oder aber gegenüber diesen chemisch inert sein.

Bevorzugte physikalisch trocknende, das heißt chemisch gegenüber den anderen Lackbestandteilen inerte Bindemittel sind zum Beispiel, Celluloseacetobutyrat ("CAB"), Polyamide oder Polyvinylbutyral.

Unter "aliphatischen Polyisocyanaten" der Komponente (b) werden Verbindungen mit mindestens zwei freien, vorzugsweise mindestens drei freien, das heißt bei Raumtemperatur (25°C) nicht blockierten Isocyanatgruppen im Molekül verstanden. Unter dem Begriff der aliphatischen Polyisocyanate werden auch Dimere, Trimere und Polymere der aliphatischen Polyisocyanate verstanden. Beispiele hierfür sind Dimere, Trimere und Polymere von Hexamethylendiisocyanat (HDI), so beispielsweise dessen Uretdione und insbesondere dessen Isocyanurate.

Das erfindungsgemäße Beschichtungsmittel kann auch weitere Vernetzer neben den oben genannten aliphatischen Polyisocyanaten enthalten. Insbesondere können neben den aliphatischen Polyisocyanaten auch cycloaliphatische Polyisocyanate, wie insbesondere Isophorondiisocyanat (IPDI) und dessen Dimere, Trimere und Polymere oder Cyclohexan(bis-alkylisocyanat) sowie deren Dimere, Trimere und Polymere eingesetzt werden. Der Einsatz aromatischer Polyisocyanate hingegen ist weniger bevorzugt, da Beschichtungen, die aus Beschichtungsmittel erhalten werden, die aromatische Polyisocyanate enthalten, zur Vergilbung neigen. Daher werden in einer besonders bevorzugten Ausführungsform der Erfindung keine aromatischen Polyisocyanate im Beschichtungsmittel eingesetzt.

Ganz besonders bevorzugt werden als aliphatische unblockierte Polyisocyanate die Trimere des HDI, wie sie beispielsweise als Basonat HI 100 von der BASF SE (Ludwigshafen, Deutschland), als Desmodur® N 3300 und Desmodur® XP 2410 von der Bayer Material Science AG (Leverkusen, Deutschland), oder als Tolonate ® HDT und HDB von Perstorp AB in Perstorp, Schweden, sowie ähnliche Produkte der Asahi Kasei Chemicals, Kawasaki, Japan, Handelsnamen Duranate® TLA, Duranate® TKA oder Duranate® MHG erhältlich sind, eingesetzt.

In untergeordneten Mengen können auch von den oben genannten Polyisocyanaten (b) verschiedene Vernetzer eingesetzt werden. Hierbei sind insbesondere solche vorteilhaft, die im gleichen Temperaturbereich Härtungsreaktionen mit den Bindemitteln eingehen, wie die aliphatischen Polyisocyanate (b). Als derartige Vernetzer sind beispielsweise Silylgruppen-haltige Komponenten wie sie in WO 2008/074489, WO 2008/074490, WO 2008/074491 genannt werden, geeignet.

Die Glyceroldiester-Komponente (c) ist in den erfindungsgemäßen Beschichtungsmittelzusammensetzungen vorzugsweise in einer Menge von 2 bis 20 Gew.-%, besonders bevorzugt in einer Menge von 3 bis 18 Gew.-%, ganz besonders bevorzugt in einer Menge von 5 bis 15 Gew.-% oder besser noch 8 bis 15 Gew.-%, bezogen auf das Gesamtgewicht der Komponenten (a) und (c) in der Beschichtungsmittelzusammensetzung enthalten. Liegt der Anteil der Glyceroldiester-Komponente (c) unterhalb 2 Gew.-% bezogen auf das Gesamtgewicht der Komponenten (a) plus (c), so ist die erfindungsgemäße Wirkung meist gering. Liegt der Anteil der Glyceroldiester-Komponente (c) oberhalb 20 Gew.-% bezogen auf das Gesamtgewicht der Komponenten (a) plus (c), so resultieren daraus häufig unzureichend vernetzte Filme die mangelnde Beständigkeiten besitzen.

Neben den zwingend im Beschichtungsmittel enthaltenen Komponenten (a), (b) und (c) können wie oben bereits erwähnt weitere von diesen verschiedene Vernetzer, Bindemittel oder Reaktivverdünner im Beschichtungsmittel enthalten sein. Insbesondere können die Zusammensetzungen auch Lösemittel enthalten. Als Lösemittel eignen sich alle lacktypischen Lösemittel wie insbesondere aromatische Kohlenwasserstoffe oder Butylacetat.

Darüber hinaus können die Beschichtungsmittel auch weitere von den Komponenten (a), (b) und (c) verschiedene lacktypische Additive, wie beispielsweise die Vernetzungsreaktion(en) katalysierende Katalysatoren, Lichtschutzmittel, Konservierungsmittel, Verlaufadditive, Antiablaufmittel (zum Beispiel sogenannte "Sag Control Agents"), Netzmittel, Mattierungsmittel, Farbstoffe, Pigmente oder Füllstoffe enthalten.

Vorzugsweise ist die erfindungsgemäße Beschichtungsmittelzusammensetzung ein Klarlack, das heißt sie ist frei beziehungsweise im Wesentlichen frei von nicht transparenten Pigmenten und Füllstoffen.

Besonders bevorzugt wird die erfindungsgemäße Beschichtungsmittelzusammensetzung bei einer Mehrschichtlackierung als oberste Lackschicht eingesetzt. Ganz besonders bevorzugt wird sie in der Automobilkarosserielackierung oder der Lackierung von Automobilkarosserieteilen als Klarlackschicht aufgebracht. Da die erfindungsgemäßen Zusammensetzungen bereits bei niedrigen Temperaturen (in der Regel bereits unter 100°C) chemisch härten, sind diese ganz besonders bevorzugt in der Automobilreparaturlackierung einsetzbar.

Die erfindungsgemäße Beschichtungsmittelzusammensetzung wird vorzugsweise kurz vor deren Applikation durch Mischen der Komponenten hergestellt, da eine Vernetzungsreaktion zwischen den freien Isocyanatgruppen des aliphatischen Polyisocyanats (b) und den im polymeren Polyol (a) und den im Glyceroldiester (c) vorhandenen Hydroxylgruppen bereits bei Raumtemperatur erfolgen kann. Im Allgemeinen unproblematisch ist eine Vorvermischung der polymeren Polyole (a) mit dem oder den Glyceroldiestern (c). In jedem Fall sollten die gegenüber einander reaktiven Bestandteile der Beschichtungsmittelzusammensetzung erst kurz vor der Applikation des Beschichtungsmittels vermischt werden, um eine möglichst lange Verarbeitbarkeit zu gewährleisten.

Weiterer Gegenstand der vorliegenden Erfindung ist eine Mehrschichtlackierung die mindestens zwei Schichten, vorzugsweise mindestens drei Schichten umfasst. Die Schichten sind auf einem grundierten oder ungrundierten Substrat angeordnet, wobei die oberste Schicht aus einer erfindungsgemäßen Beschichtungsmittelzusammensetzung gebildet ist. Ist das Substrat grundiert, so ist die Grundierung vorzugsweise eine Elektrotauchlackgrundierung, insbesondere eine kathodische Elektrotauchlacklackierung. Der Grundierung kann insbesondere auch eine Phosphatierung vorausgehen.

Auf dem grundierten oder ungrundierten Substrat kann beispielsweise ein herkömmliches Füllerbeschichtungsmittel aufgebracht sein. Auf dem Füllerbeschichtungsmittel - sofern dieses vorhanden ist - können ein oder mehrere Basislackierungen aufgebracht werden. Im Falle der Automobilreparaturlackierung handelt es sich üblicherweise um rein physikalisch trocknende Basislackierungen oder thermisch, mittels eines Vernetzers härtende Basislackierungen oder solche die chemisch bei moderaten Temperaturen (Raumtemperatur bis 100°C) und aktinisch oder nur aktinisch härten. Es besteht auch die Möglichkeit Füllerbeschichtungsmittel mit den Eigenschaften einer Basislackierung auszustatten oder umgekehrt eine Basislackierung mit Füllereigenschaften, so dass es ausreichen kann, nur eine Füllerschicht oder nur eine Basislackschicht aufzubringen. Üblicherweise wird ein Füllerbeschichtungsmittel als Füllerschicht und mindestens eine Basislackzusammensetzung als Basislackschicht aufgebracht. Als Füllerbeschichtungsmittel und als Basislackzusammensetzung eignen sich alle handelsüblichen Füller oder Basislacke, insbesondere solche, wie sie in der Automobilreparaturlackierung eingesetzt werden. Als letzte Schicht ist schließlich eine erfindungsgemäße Beschichtungszusammensetzung als Decklack, vorzugsweise als transparenter Decklack (Klarlack) aufgebracht.

Weiterer Gegenstand der Erfindung ist dementsprechend auch ein Verfahren zur Herstellung einer erfindungsgemäßen Mehrschichtlackierung, umfassend die Schritte:
(i) Aufbringen eines Füllerbeschichtungsmittels auf ein unbehandeltes oder vorbehandeltes Substrat und Aufbringen mindestens einer Basislackzusammensetzung darauf, oder
(ii) Aufbringen mindestens einer Basislackzusammensetzung auf ein unbehandeltes oder vorbehandeltes Substrat; und anschließendes
(iii) Aufbringen mindestens einer erfindungsgemäßen Klarlackzusammensetzung, gefolgt von einer
(iv) Härtung der Mehrschichtlackierung bei einer Temperatur bis maximal 100°C.

Als Füllerbeschichtungsmittel und Basislackzusammensetzung können herkömmliche, handelsübliche Füller und Basislacke eingesetzt werden. Insbesondere sind solche geeignet wie sie in der Automobilreparaturlackierung eingesetzt werden.

Das Aufbringen der einzelnen Schichten erfolgt nach den üblichen, dem Durchschnittsfachmann geläufigen Lackierverfahren. Vorzugsweise werden die Zusammensetzungen bzw. Beschichtungsmittel durch Sprühen pneumatisch und/oder elektrostatisch aufgebracht.

Die Schritte (i), (ii) und (iii) erfolgen vorzugsweise Nass-in-Nass. Vor dem Aufbringen der Basislackzusammensetzung oder Basislackzusammensetzungen kann der Füller nur bei Raumtemperatur abgelüftet werden oder jedoch auch bei erhöhter Temperatur von vorzugsweise maximal 100°C, besonders bevorzugt 30 bis 80°C und ganz besonders bevorzugt 40 bis 60°C getrocknet werden. Eine Trocknung kann auch durch IR-Bestrahlung erfolgen.

Was für die Füllerlackierung hinsichtlich des Ablüftens und/oder Trocknens und der diesbezüglichen Trocknungstemperaturen gilt, das gilt in gleicher Weise für die Basislackschicht beziehungsweise Basislackschichten.

Anstelle eines Naß-in-Naß-Auftrags ist auch eine Härtung der Füller- und/oder Basislack-Schicht(en) vor dem Auftrag der Schicht in Schritt (iii) möglich. Die herkömmlichen Füller und/oder Basislacke lassen sich thermisch, mit aktinischer Strahlung oder kombiniert thermisch und mit aktinischer Strahlung härten.

Die Härtung der Klarlackschicht erfolgt ebenfalls bei Temperaturen bis maximal 100°C, besonders bevorzugt bei Temperaturen von Raumtemperatur bis maximal 80°C, und ganz besonders bevorzugt bei Temperaturen von 30 bis 60°C. Der Härtung kann auch ein Ablüften vorangehen.

Ein weiterer Gegenstand der vorliegenden Erfindung ist ein Substrat, auf dem eine erfindungsgemäße Mehrschichtlackierung aufgebracht ist.

Als Substratmaterial eignen sich insbesondere metallische Substrate, wie beispielsweise Automobilkarosserien oder Automobilkarosserieteile, aber auch Kunststoffsubstrate, wie sie insbesondere bei der 2-Komponenten-Serienlackierung von Kunststoffteilen eingesetzt werden.

Im Folgenden soll die Erfindung anhand von Beispielen illustriert werden.

### BEISPIELE

Für die Durchführung der folgenden Experimente wurden Reagentien und Lösemittel in technischer Reinheit von verschiedenen Herstellern eingesetzt. Cardura® E10 und Versaticsäure wurden von der Firma Hexion (Louvain-la-Neuve, Belgien) bezogen. Zur Durchführung der Größenausschluß GPC wurde die "Isocratic Mode Pump Waters 515" und die Säulen HR5E (linear) und HR2 (500) in Reihe eingesetzt (Firma Waters, Eschborn, Deutschland und PSS Polymer Standard Services Mainz, Deutschland). Als Polystyrolstandard diente "Calibration Polystyrene PS2" der Firma Polymer Laboratories (Darmstadt, Deutschland) (580 bis 377400 Da). Die Viskosität nach Gardner wurde mit Standard Gardner Tubes (Firma Byk Gardner, Geretsried, Deutschland) und die Brookfield-Viskosität mit dem Gerät CAP 2000 (Brookfield E.L.V. GmbH, Lorch, Deutschland) bestimmt. DOI-Messungen wurden mit dem Gerät Wave-Scan DOI 4816 der Firma Byk Gardner (Geretsried, Deutschland) durchgeführt.

### Beispiel 1: Herstellung eines erfindungsgemäßen Glyceroldiesters

Einem 5-Liter Reaktionsbehälter, ausgestattet mit einem mechanischen Rührer und Rückflußkühler, wurden 1241,1 g (7,25 mol) Versaticsäure und 1758,9 g (7,10 mol) Cardura® E10 zugegeben. Es wurde bei einer Rührgeschwindigkeit von 150 Umdrehungen pro Minute auf 150°C erhitzt. Der Reaktionsfortschritt wurde durch Bestimmung der Säurezahl verfolgt. Nach etwa einer Stunde war die Umsetzung vollständig abgeschlossen. Es wurden 3000 g einer klaren, leicht gelblichen Flüssigkeit erhalten. Die Viskosität nach Gardner betrug I-K. Die Viskosität nach Brookfield (Cone Plate 3, 200 Umdrehungen pro Minute, 23°C) betrug ca. 325 mPas. Die Säurezahl betrug ca. 5 mg KOH/g. Die Farbzahl (APHA) betrug: 40. Das zahlenmittlere und gewichtsmittlere Molekulargewicht wurde mit GPC gegen einen Polystryrolstandard unter Nutzung eines Brechungsindexdetektors bestimmt und betrug: M_{w}: 450 Dalton und Mₙ: 430 Dalton. Das Verhältnis primärer OH-Gruppen zu sekundären OH-Gruppen im Zielprodukt betrug 1:1,27 (bestimmt mittels ¹H-NMR). Das heißt, es lag eine Mischung zweier Glyceroldiester vor (nach obiger Definition betrug der Anteil an Verbindungen mit R¹ = H 44 % und der Anteil der Verbindungen mit R² = H 56 %).

### Beispiel 2: Herstellung eines erfindungsgemäßen Glyceroldiesters (in-situ)

Zunächst wurde in einem mit einem mechanischen Rührer ausgestatteten 6-Liter-Reaktionsgefäß eine Mischung aus 225, 6 g (0,91 mol) Cardura® E10 [CAS 26761-45-5], 159,2 g (0,93 mol) Versaticsäure [CAS 26896-20-8] und 680,5 g Butylacetat in Gegenwart von 5,0 g (0,01 mol) Triisodecylphosphit als Katalysator unter einer Stickstoffatmosphäre (2,5 bar) unter Rühren (150 Umdrehungen pro Minute) auf 160 °C erhitzt.

Anschließend wurden die verschiedenen Reagenzien über eine HPLC-Pumpe zugeführt: eine erste Menge Di-tert.-Butylperoxid (6,8 g, 0,05 mol) als Initiator in 30,0 g Butylacetat, anschließend, nach 15 Minuten, über einen Zeitraum von 4 Stunden bei 160°C wurde eine homogene Mischung aus 522,8 g (5,22 mol) Methylmethacrylat, 1215,8 g (11,69 mol) Styren, 30,4 g (0,24 mol) Butylacrylat, 249,2 g (1,75 mol) Butylmethacrylat, 30,4 g (0,42 mol) Acrylsäure und 990,5 g (7,61 mol) Hydroxyethylmethacrylat sowie weitere 103,0 g (0,70 mol) Di-tert.-Butylperoxid und 77,0 g Butylacetat zugeführt. Danach wurde über einen Zeitraum von 30 Minuten weitere 13,6 g (0,10 mol) Di-tert.-Butylperoxid in 20,0 g Butylacetat zugegeben, um eine vollständige Umsetzung der Monomeren zu gewährleisten. Die Reaktionsmischung wurde 100°C abgekühlt während ein Druckausgleich auf Atmosphärendruck durchgeführt wurde und mit 760,0 g Methylisobutylketon verdünnt wurde um 5000,0 g einer klaren farblosen und viskosen Lösung des Polymers zu erhalten. Der Festkörper beträgt ca. 72 % (1 g wurde 1 Stunde bei 110°C getrocknet). Das erhaltene hydroxyfunktionelle Poly(meth)acrylat besitzt eine Säurezahl von ca. 14 mg KOH/g, eine Hydroxylzahl von 140 mg KOH/g, ein zahlenmittleres Molekulargewicht Mₙ von 1920 g/mol (bestimmt mittels Gelpermeationschromatographie (GPC) gegen einen Polystyrolstandard) und ein gewichtsmittleres Molekulargewicht M_{w} von 3680 g/mol (bestimmt mittels GPC gegen einen Polystyrolstandard). Die Viskosität beträgt nach Gardner Z-Z2 und nach Brookfield (Cone Plate 3, 50 Umdrehungen pro Minute, 23°C) ca. 11200 mPas.

### Beispiel 3: Herstellung eines hydroxyfunktionellen Poly(meth)acrylats

Zunächst wurde in einem mit einem mechanischen Rührer ausgestatteten 6-Liter-Reaktionsgefäß 727,3 g Butylacetat unter einer Stickstoffatmosphäre (2,5 bar) unter Rühren (150 Umdrehungen pro Minute auf 160°C) erhitzt.

Anschließend wurden die verschiedenen Reagenzien über eine HPLC-Pumpe zugeführt: eine erste Menge Di-tert.-Butylperoxid (8,5 g, 0,06 mol) als Initiator in 30,0 g Butylacetat, anschließend, nach 15 Minuten, über einen Zeitraum von 4 Stunden bei 160°C wurde eine homogene Mischung aus 574,5 g (5,74 mol) Methylmethacrylat, 1336,0 g (12,85 mol) Styren, 33,4 g (0,26 mol) Butylacrylat, 273,9 g (1,92 mol) Butylmethacrylat, 33,4 g (0,46 mol) Acrylsäure und 1088,4 g (8,36 mol) Hydroxyethylmethacrylat sowie weitere 135,1 g (0,92 mol) Di-tert.-Butylperoxid und 113,6 g Butylacetat zugeführt. Danach wurde über einen Zeitraum von 30 Minuten weitere 17,0 g (0,12 mol) Di-tert.-Butylperoxid in 20,0 g Butylacetat zugegeben, um eine vollständige Umsetzung der Monomeren zu gewährleisten. Die Reaktionsmischung wurde 100°C abgekühlt während ein Druckausgleich auf Atmosphärendruck durchgeführt wurde und mit 680,0 g Methylisobutylketon verdünnt wurde um 5050,0 g einer klaren farblosen und viskosen Lösung des Polymers zu erhalten. Der Festkörper beträgt ca. 72 % (1 g wurde 1 Stunde bei 110°C getrocknet). Das erhaltene hydroxyfunktionelle Poly(meth)acrylat besitzt eine Säurezahl von ca. 14 mg KOH/g, eine Hydroxylzahl von 126 mg KOH/g, ein zahlenmittleres Molekulargewicht Mₙ von 2260 g/mol (bestimmt mittels Gelpermeationschromatographie (GPC) gegen einen Polystyrolstandard) und ein gewichtsmittleres Molekulargewicht M_{w} von 4750 g/mol (bestimmt mittels GPC gegen einen Polystyrolstandard). Die Viskosität beträgt nach Gardner Z1-Z3 und nach Brookfield (Cone Plate 3, 50 Umdrehungen pro Minute, 23°C) ca. 6350 mPas.

### Anwendungsbeispiele 1 (Vergleich) und 1A (erfindungsgemäß)

Es wurden gemäß folgender Tabelle 1 Beschichtungsmittelzusammensetzungen (Anwendungsbeispiel 1 (nicht erfindungsgemäß) und Anwendungsbeispiel 1A (erfindungsgemäß) hergestellt.

**Tabelle 1**

| | Anwendungsbeispiel 1 (Gew.-Teile) | Anwendungsbeispiel 1A (Gew.-Teile) |
|---|---|---|
| Zusammensetzung aus Beispiel 2 (Glyceroldiester inkl. Poly(meth)acrylat) | - | 78 |
| Zusammensetzung aus Beispiel 3 (nur Poly(meth)acrylat) | 78 | - |
| Benzoesäure | 1,5 | 1,5 |
| Dibutylzinndilaurat | 0,18 | 0,18 |
| Lichtschutzmittel aus HALS und UV-Absorber | 2,2 | 2,2 |
| polyestermod ifiziertes Polymethylalkylsiloxan | 0,3 | 0,3 |
| Lösemittelmischung (aromatische Lösemittel und Ester) | 16,2 | 16,2 |
| Festkörper (1g, 1 Stunde, 110°C) | 60% | 60% |
| Härter 929,33 (Glasurit GmbH) | 49,2 | 49,2 |
| Verdünnung 352-91 (Glasurit GmbH) | 8,5 | 8,5 |
| Festkörper (1g, 1 Stunde, 110°C) | 56% | 56% |

Der Klarlack des nicht-erfindungsgemäßen Anwendungsbeispiels 1 besaß eine Auslaufviskosität nach der DIN4-Becher-Methode von 20s. Klarlack 1A (erfindungsgemäß) wurde mit gleicher Menge Verdünnung versehen, zeigte dabei eine Auslaufviskosität von nur 18s im DIN4-Becher. Die Bestimmung des Feststoffgehaltes (1g, 110°C, 1h) ergab für beide Klarlackmischungen 56%.

Mit einem Coil-primer vorbeschichtete Bleche von 40 x 60 cm wurden zunächst mit einem handelsüblichen Füller (Glasurit 285-31) und einem Wasserbasislack (Glasurit, Reihe 90, tiefschwarz) beschichtet. Der Basislack wurde für 10 Minuten bei 60°C im Umluftofen vorgetrocknet. Anschließend wurden die Klarlacke der Anwendungsbeispiele 1 und 1A mittels pneumatischer Sprühapplikation (SATA HVLP) mit einer resultierenden Trockenschichtdicke von 61 Mikrometer (Klarlack 1) bzw. 59 Mikrometer (Klarlack 1A) aufgetragen. Nach einer Ablüftphase bei RT für 10 Minuten, wobei dazu die Tafeln senkrecht aufgehängt werden, werden die Tafeln senkrecht hängend bei 60°C für 30 Minuten getrocknet.

Anschließend wurde das Erscheinungsbild (Appearance) bestimmt. Bei nahezu identischer Trockenschichtdicke und nahezu identischer Abbildungsschärfe (Distinctness of Image; DOI) (84,3 (Anwendungsbeispiel 1) bzw. 84,2 (Anwendungsbeispiel 1A), sind die Wavescan-Ergebnisse deutlich unterschiedlich. Die Beschichtung aus dem erfindungsgemäßen Beschichtungsmittel besitzt sowohl im Long-Wave- als auch im Short-Wave-Bereich deutlich bessere Werte als die Beschichtung aus dem nicht erfindungsgemäßen Beschichtungsmittel (Long-Wave (erfindungsgemäß): 20,7, Long-Wave (nicht erfindungsgemäß): 27,1; Short-Wave (erfindungsgemäß): 15,1, Short-Wave (nicht erfindungsgemäß): 18,6). Hierdurch wird ein deutlich besserer Verlauf der erfindungsgemäßen Lackierungen bei nahezu gleicher, sogar etwas niedrigerer Schichtdicke belegt. Auch besitzt der erfindungsgemäße Klarlack, bei gleichem Festkörper den Vorteil einer geringeren Viskosität.

## Patentansprüche

1. Verwendung von mindestens einem Glyceroldiester der allgemeinen Formel (I) 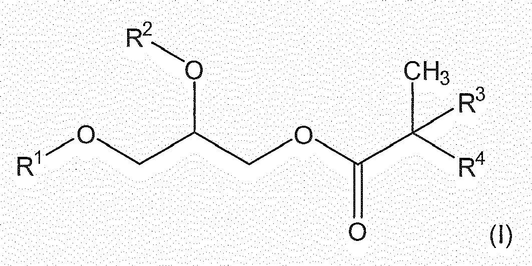 worin
einer der beiden Reste R¹ oder R² für Wasserstoff steht und der nicht für Wasserstoff stehende der beiden Reste R¹ und R² für einen Rest 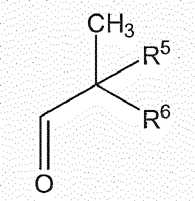 steht, und
die Reste R³, R⁴, R⁵ und R⁶ unabhängig voneinander für einen Alkylrest mit 1 bis 10 Kohlenstoffatomen stehen,
mit der Maßgabe, dass die Reste R³ und R⁴ gemeinsam mindestens 4 Kohlenstoffatome enthalten und die Reste R⁵ und R⁶ gemeinsam mindestens 4 Kohlenstoffatome enthalten, als Reaktivverdünner in Beschichtungsmittelzusammensetzungen, Klebstoffen und Dichtungsmitteln.

2. Verwendung gemäß Anspruch 1, wobei der Glyceroldiester in einer Beschichtungsmittelzusammensetzung als Reaktivverdünner verwendet wird und es sich bei der Beschichtungsmittelzusammensetzung um einen Klarlack handelt.

3. Beschichtungsmittelzusammensetzung enthaltend
(a) mindestens ein polymeres Polyol gewählt aus der Gruppe bestehend aus Poly(meth)acrylat-Polyolen, Polyester-Polyolen, Polyurethan-Polyolen und Polysiloxan-Polyolen,
(b) mindestens ein aliphatisches Polyisocyanat und
(c) mindestens einen Glyceroldiester der allgemeinen Formel (I) gemäß Anspruch 1.

4. Beschichtungsmittelzusammensetzung gemäß Anspruch 3, wobei (a) mindestens ein Poly(meth)acrylat-Polyol umfasst.

5. Beschichtungsmittelzusammensetzung gemäß Anspruch 3 oder 4, wobei die Hydroxylzahl der Komponente (a) um nicht mehr als 20 % von der Hydroxylzahl der in der Beschichtungsmittelzusammensetzung eingesetzten Glyceroldiester-Komponente (c) abweicht und/oder der Anteil der Glyceroldiester-Komponente (c) 2 bis 20 Gew.-% bezogen auf das Gesamtgewicht der Komponenten (a) plus (c) beträgt.

6. Mehrschichtlackierung, die mindestens zwei Schichten umfasst, die auf einem Substrat angeordnet sind und die **dadurch gekennzeichnet ist, dass** die oberste Schicht der Schichten aus einer Beschichtungsmittelzusammensetzung gemäß einem oder mehreren der Ansprüche 3 bis 5 besteht.

7. Verfahren zur Herstellung einer Mehrschichtlackierung, umfassend die Schritte:
(i) Aufbringen eines Füllerbeschichtungsmittels auf ein unbehandeltes oder vorbehandeltes Substrat und Aufbringen mindestens einer Basislackzusammensetzung darauf, oder
(ii) Aufbringen mindestens einer Basislackzusammensetzung auf ein unbehandeltes oder vorbehandeltes Substrat; und anschließendes
(iii) Aufbringen mindestens einer Klarlackzusammensetzung, wobei die Klarlackzusammensetzung eine Beschichtungszusammensetzung nach einem oder mehreren der Ansprüche 3 bis 5 ist, gefolgt von einer
(iv) Härtung der Mehrschichtlackierung bei einer Temperatur von bis zu maximal 100°C.

8. Verfahren nach Anspruch 7, wobei Schritt (iv) bei einer Temperatur von 30 bis 60°C durchgeführt wird.

9. Substrat, **dadurch gekennzeichnet, dass** auf diesem eine Mehrschichtlackierung gemäß Anspruch 6 oder gemäß einem Verfahren nach einem der Ansprüche 7 oder 8 aufgebracht ist.

## Claims

1. Use of at least one glycerol diester of the general formula (I) 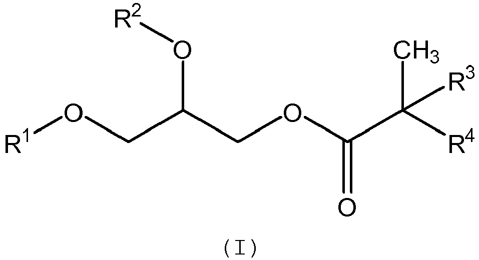 in which
one of the two radicals R¹ or R² is hydrogen and the radical of the two radicals R¹ and R² that is not hydrogen is a radical 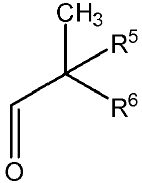 and
the radicals R³, R⁴, R⁵ and R⁶ independently of one another are an alkyl radical having 1 to 10 carbon atoms,
with the proviso that the radicals R³ and R⁴ together contain at least 4 carbon atoms and the radicals R⁵ and R⁶ together contain at least 4 carbon atoms, as reactive diluents in coating material compositions, adhesives and sealants.

2. Use according to claim 1, where the glycerol diester is used in a coating material composition as reactive diluent and the coating material composition is a clearcoat material.

3. Coating material composition comprising
(a) at least one polymeric polyol selected from the group consisting of poly(meth)acrylate polyols, polyester polyols, polyurethane polyols and polysiloxane polyols,
(b) at least one aliphatic polyisocyanate and
(c) at least one glycerol diester of the general formula (I) according to claim 1.

4. Coating material composition according to claim 3, where (a) comprises at least one poly(meth)acrylate polyol.

5. Coating material composition according to claim 3 or 4, where the hydroxyl number of component (a) differs by not more than 20% from the hydroxyl number of the glycerol diester component (c) used in the coating material composition, and/or the fraction of the glycerol diester component (c) is 2% to 20% by weight, based on the total weight of components (a) plus (c).

6. Multicoat paint system comprising at least two coats which are disposed on a substrate, **characterized in that** the uppermost coat of the coats is composed of a coating material composition according to one or more of claims 3 to 5.

7. Method for producing a multicoat paint system, comprising the following steps:
(i) applying a primer-surfacer coating material to an untreated or pretreated substrate and applying at least one basecoat composition thereto, or
(ii) applying at least one basecoat composition to an untreated or pretreated substrate; and subsequently
(iii) applying at least one clearcoat composition, the clearcoat composition being a coating composition according to one or more of claims 3 to 5, followed by
(iv) curing of the multicoat paint system at a temperature of up to 100°C maximum.

8. Method according to claim 7, where step (iv) is carried out at a temperature of 30 to 60°C.

9. Substrate **characterized in that** applied thereto is a multicoat paint system according to claim 6 or according to a method according to either of claims 7 and 8.

## Revendications

1. Utilisation d'au moins un diester de glycérol de formule générale (I) 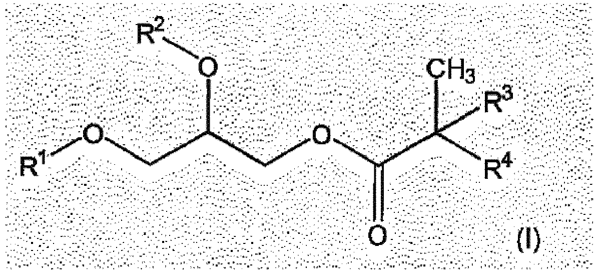 dans laquelle
un des deux radicaux R¹ ou R² représente l'hydrogène et celui des deux radicaux R¹ et R² qui ne représente pas l'hydrogène représente un radical 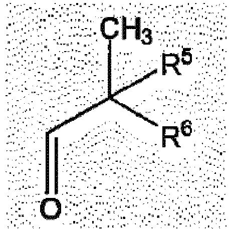 et
les radicaux R³, R⁴, R⁵ et R⁶ représentent indépendamment les uns des autres un radical alkyle de 1 à 10 atomes de carbone,
à condition que les radicaux R³ et R⁴ contiennent ensemble au moins 4 atomes de carbone, et que les radicaux R⁵ et R⁶ contiennent ensemble au moins 4 atomes de carbone, en tant que diluant réactif dans des compositions d'agent de revêtement, des adhésifs et des agents d'étanchéité.

2. Utilisation selon la revendication 1, dans laquelle le diester de glycérol est utilisé dans une composition d'agent de revêtement en tant que diluant réactif, et la composition d'agent de revêtement est un vernis transparent.

3. Composition d'agent de revêtement, contenant :
(a) au moins un polyol polymère choisi dans le groupe constitué par les poly(méth)acrylate-polyols, les polyester-polyols, les polyuréthane-polyols et les polysiloxane-polyols,
(b) au moins un polyisocyanate aliphatique et
(c) au moins un diester de glycérol de formule générale (I) selon la revendication 1.

4. Composition d'agent de revêtement selon la revendication 3, dans laquelle (a) comprend au moins un poly(méth)acrylate-polyol.

5. Composition d'agent de revêtement selon la revendication 3 ou 4, dans laquelle l'indice hydroxyle du composant (a) ne diffère pas de plus de 20 % de l'indice hydroxyle du composant diester de glycérol (c) utilisé dans la composition d'agent de revêtement et/ou la proportion du composant diester de glycérol (c) est de 2 à 20 % en poids par rapport au poids total des composants (a) plus (c).

6. Vernissage multicouche, qui comprend au moins deux couches, qui sont agencées sur un substrat, et qui est **caractérisé en ce que** la couche la plus supérieure des couches est constituée d'une composition d'agent de revêtement selon une ou plusieurs des revendications 3 à 5.

7. Procédé de fabrication d'un vernissage multicouche, comprenant les étapes suivantes :
(i) l'application d'un agent de revêtement de remplissage sur un substrat non traité ou prétraité et l'application d'au moins une composition de vernis de base sur celui-ci, ou
(ii) l'application d'au moins une composition de vernis de base sur un substrat non traité ou prétraité, puis
(iii) l'application d'au moins une composition de vernis transparent, la composition de vernis transparent étant une composition de revêtement selon une ou plusieurs des revendications 3 à 5, suivie par
(iv) le durcissement du vernissage multicouche à une température de jusqu'à 100 °C au plus.

8. Procédé selon la revendication 7, dans lequel l'étape (iv) est réalisée à une température de 30 à 60 °C.

9. Substrat, **caractérisé en ce qu'**un vernissage multicouche selon la revendication 6 ou fabriqué par un procédé selon l'une quelconque des revendications 7 ou 8 est appliqué sur celui-ci.
